# EUROPEAN PATENT APPLICATION

(11) **EP 4 643 799 A1**
(43) Date of publication of application: **05.11.2025**
(21) Application number: 22970057.0
(22) Date of filing: 27.12.2022
(51) Int. Cl.: A61B 17/68

(54) **BONE PLATE AND BONE PLATE KIT**

(71) Applicant: Olympus Terumo Biomaterials Corp., Tokyo 151-0073 (JP)
(72) Inventor: GOROKU, Yuichiro, Tokyo 151-0073 (JP)
(74) Representative: Nederlandsch Octrooibureau
(86) International application number: PCT/JP2022/048221
(87) International publication number: WO 2024/142263

(57) **Abstract**

A bone plate (1) includes: a shaft portion (2) extending in a longitudinal direction (X); and a head portion (3) disposed on a proximal side of the shaft portion (2) and having four screw holes arranged in a quadrangular shape. The four screw holes include first and second screw holes (5a, 5c) arranged in the lateral direction (Y), and a third screw hole (5b) and a fourth screw hole (5d) located between the first screw hole (5a) and the second screw hole (5c) in the lateral direction (Y) and arranged further on the proximal side and distal side with respect to the first and second screw holes (5a, 5c), respectively.

## Description

### {Technical Field}

The present invention relates to a bone plate and a bone plate kit.

### {Background Art}

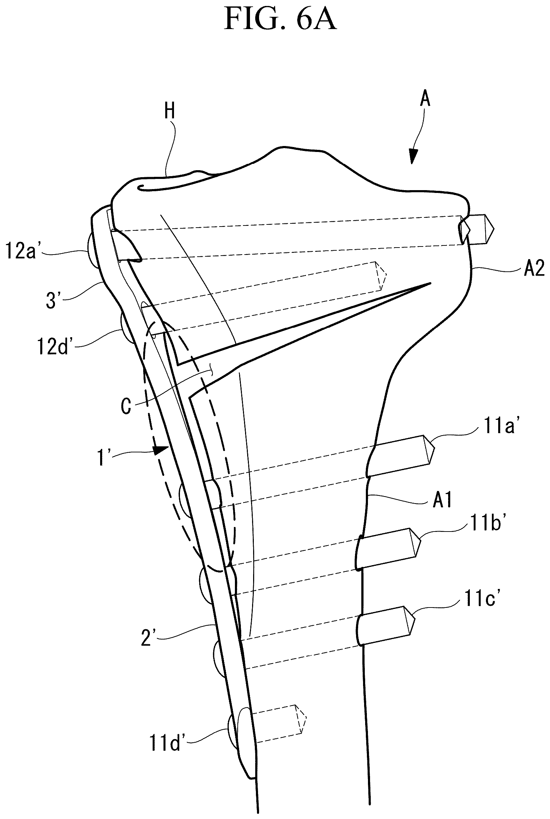

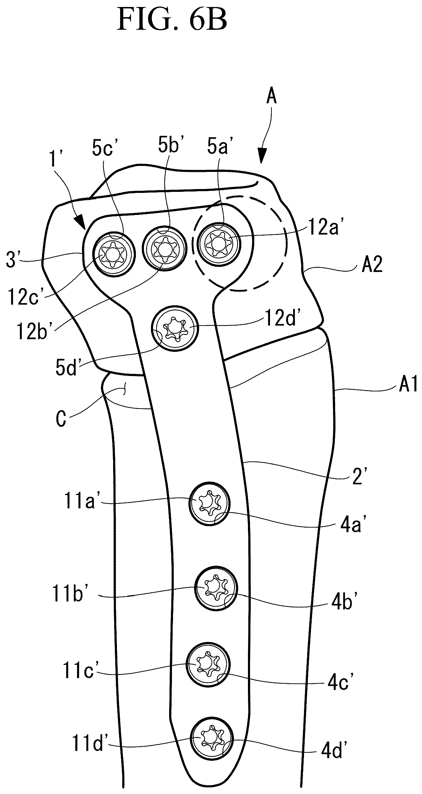

Conventionally, a T-shaped bone plate for opening wedge high tibial osteotomy (OWHTO) is known (for example, see PTL 1). As shown in Figs. 6A and 6B, a T-shaped bone plate 1' has a long shaft portion 2' disposed on the diaphyseal A1, along the longitudinal direction of the tibia A, and a short head portion 3' disposed on the epiphyseal A2, along the anteroposterior direction of the tibia A.

### {Citation List}

### {Patent Literature}

PTL 1: Japanese Patent No. 5777047

### {Summary of Invention}

### {Technical Problem}

The tibia of a small patient is small compared to the typical size tibia. When applied to such a small tibia, the conventional T-shaped bone plate 1' has the following disadvantages. First, the anterior portion of the head portion 3' (the encircled portion in Fig. 6B) is lifted from the surface of the epiphyseal A2, creating a space between the head portion 3' and the epiphyseal A2. Second, the shaft portion 2' is too long, and, for example, the distal end of the shaft portion 2' is disposed near the center of the diaphyseal A1. Third, due to the shaft portion 2' being too long, the bone plate 1' is lifted from the medial surface of the tibia A, creating a space between the bone plate 1' and the tibia A (see the encircled region in Fig. 6A). Because the bone plate 1' lifted from the surface of the tibia A may affect the surrounding tissue such as the skin, it is desirable that the gap between the bone plate 1' and the tibia A be small.

The present invention has been made in view of the above-described circumstances, and an object thereof is to provide a bone plate and a bone plate kit that fit well to the shape of the surface of the tibia of a small patient.

### {Solution to Problem}

An aspect of the present invention is a bone plate to be disposed on a medial surface of a tibia, the bone plate having a shape of a band having a longitudinal direction arranged along a longitudinal direction of the tibia and a lateral direction arranged along an anteroposterior direction of the tibia, having a proximal side and a distal side corresponding to a proximal side and a distal side of the tibia, respectively, and including: a shaft portion extending in the longitudinal direction; and a head portion disposed on a proximal side of the shaft portion. The head portion has four screw holes arranged in a quadrangular shape, and the four screw holes include a first screw hole and a second screw hole arranged in the lateral direction with a space therebetween, a third screw hole located between the first screw hole and the second screw hole in the lateral direction and disposed further on the proximal side than the first and second screw holes, and a fourth screw hole located between the first screw hole and the second screw hole in the lateral direction and disposed further on the distal side than the first and second screw holes.

Another aspect of the present invention is a bone plate kit including: the above-described bone plate; and four bone screws to be inserted into the four screw holes. Center axes of the four screw holes are inclined with respect to each other in directions toward each other as the center axes are farther from the bone plate in insertion directions of the bone screws. At least three of the four bone screws passing through the four screw holes contact each other at distal ends thereof to form a space truss structure.

### {Brief Description of Drawings}

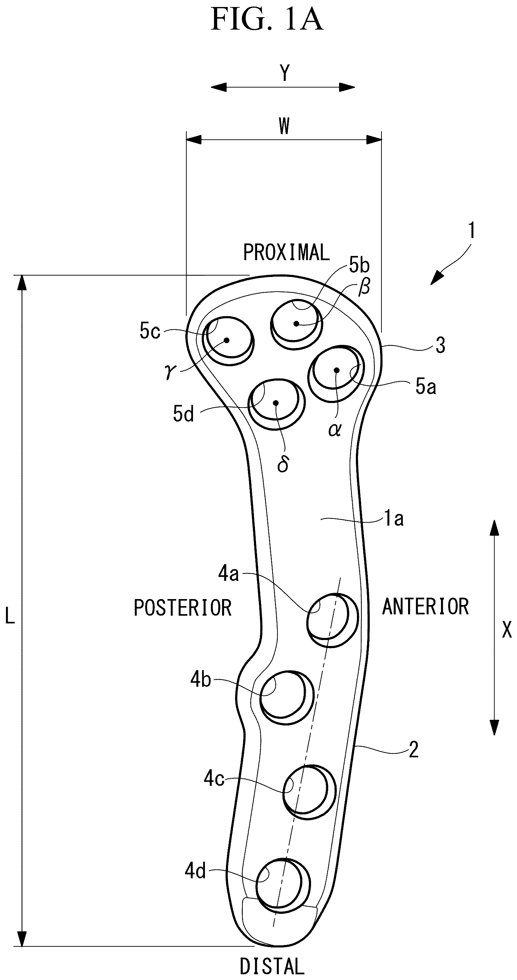

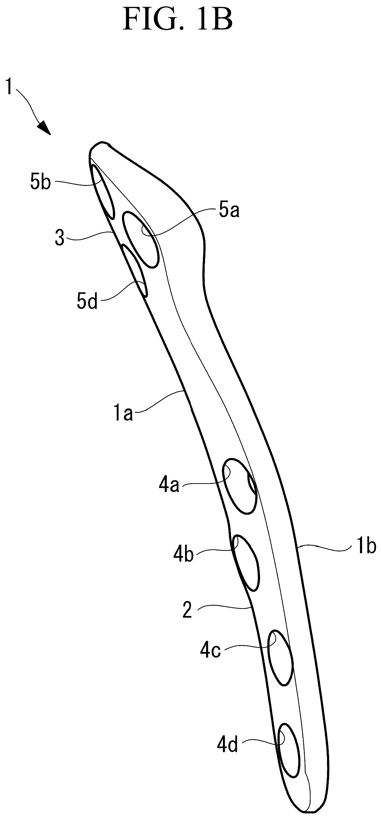

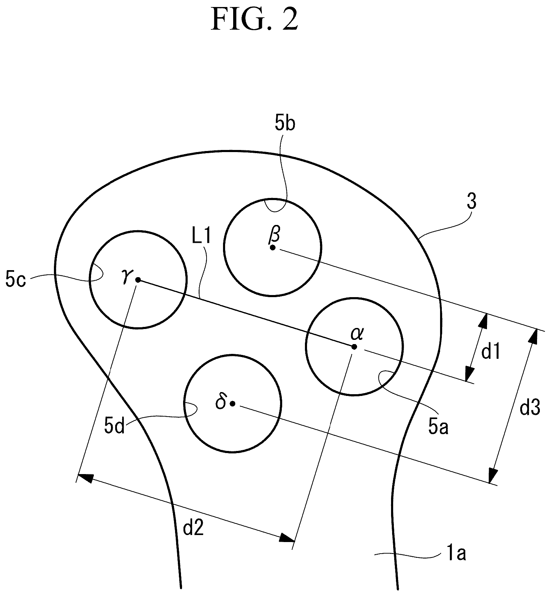

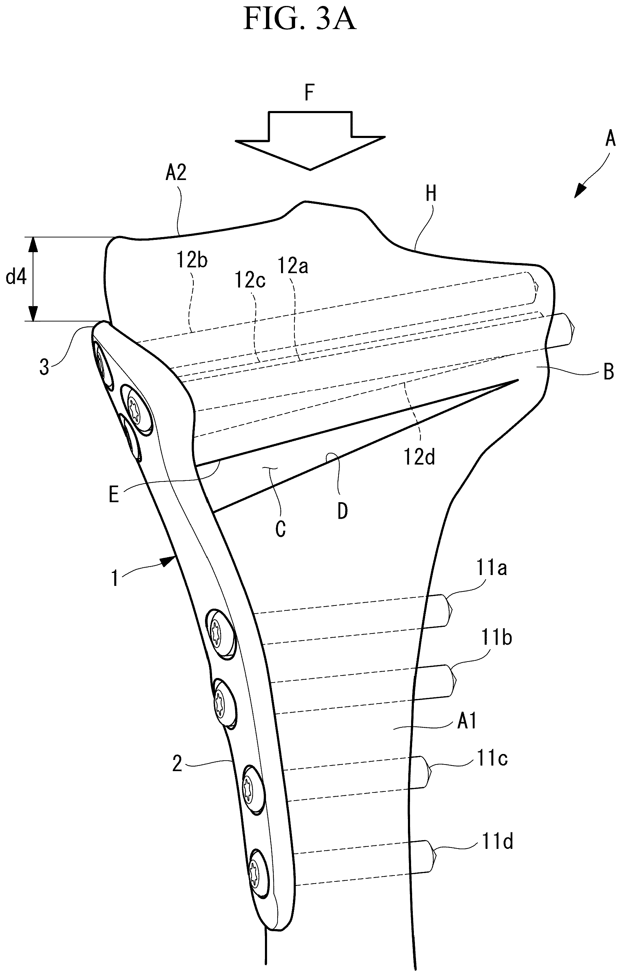

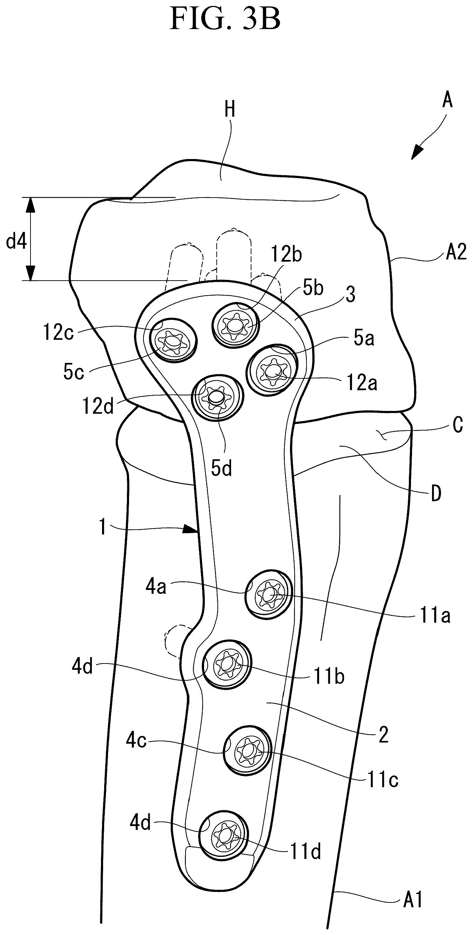

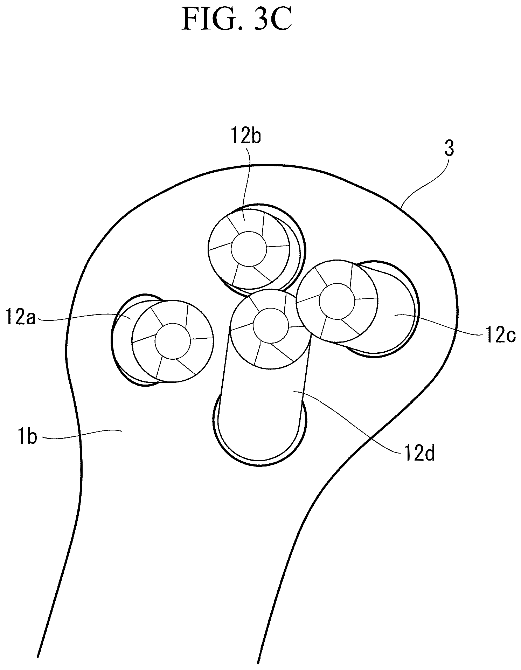

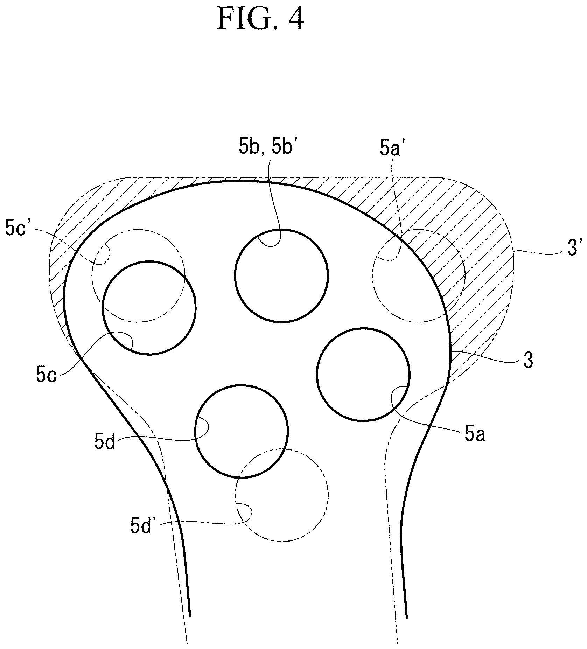

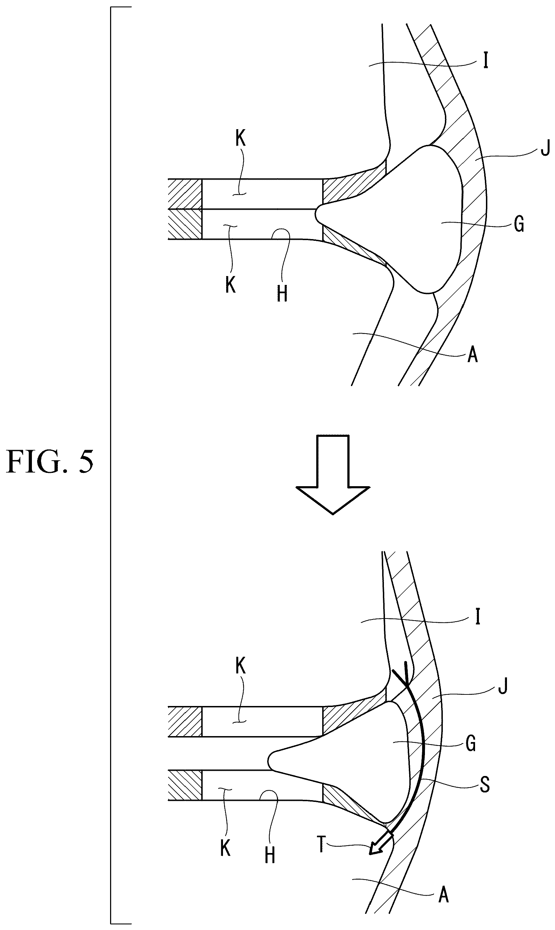

{Fig. 1A} Fig. 1A is a front view of a bone plate according to an embodiment.
{Fig. 1B} Fig. 1B is a side view of the bone plate in Fig. 1A, as viewed from the anterior side.
{Fig. 2} Fig. 2 is an enlarged front view of a head portion of the bone plate in Fig. 1A.
{Fig. 3A} Fig. 3A shows the tibia to which the bone plate in Fig. 1A is fixed, as viewed from the anterior side.
{Fig. 3B} Fig. 3B shows the tibia to which the bone plate in Fig. 1A is fixed, as viewed from the medial side.
{Fig. 3C} Fig. 3C shows an assembly of the bone plate in Figs. 3A and 3B and bone screws, as viewed from the lateral side of the tibia.
{Fig. 4} Fig. 4 shows the head portion of the bone plate in Fig. 1A and the head portion of the conventional bone plate superimposed on each other.
{Fig. 5} Fig. 5 illustrates a meniscus centralization technique.
{Fig. 6A} Fig. 6A shows the tibia to which a conventional bone plate is fixed, as viewed from the anterior side.
{Fig. 6B} Fig. 6B shows the tibia to which the conventional bone plate is fixed, as viewed from the medial side.

### {Detailed Description of the Invention}

Hereinbelow, a bone plate and a bone plate kit according to an embodiment of the present invention will be described with reference to the drawings.

As shown in Figs. 3A and 3B, the bone plate kit according to this embodiment is for opening wedge high tibial osteotomy (OWHTO), and includes a bone plate 1 to be disposed on the medial surface of the tibia A, and a plurality of bone screws 11a to 11d and 12a to 12d for fixing the bone plate 1 to the tibia A.

In OWHTO, the tibia A is cut from the medial side while leaving a hinge portion B on the lateral side, and an artificial bone or an autologous bone (not shown) is inserted into the opened wedge-shaped cut portion C. Next, the bone plate 1 is disposed on the medial surface of the tibia A across the cut portion C and is fixed to the tibia A with the bone screws 11a to 11d and 12a to 12d.

As shown in Figs. 1A and 1B, the bone plate 1 is a band-like long plate having a longitudinal direction X and a lateral direction Y. The longitudinal direction X is a direction along the longitudinal direction of the tibia A. The lateral direction Y is a direction intersecting the longitudinal direction X and along the anteroposterior direction of the tibia A. The bone plate 1 is curved around an axis extending in the longitudinal direction X, so that a rear side 1b to be in contact with the surface of the tibia A conforms to the curved shape of the medial surface of the tibia A.

The bone plate 1 has the distal side, the proximal side, the anterior side, and the posterior side corresponding to the distal side, the proximal side, the anterior side, and the posterior side of the tibia A, respectively (see Fig. 1A). The distal side is one side in the longitudinal direction X, and the proximal side is the opposite side from the distal side in the longitudinal direction X. The anterior side is one side in the lateral direction Y, and the posterior side is the opposite side from the anterior side in the lateral direction Y. The bone plate 1 shown in the drawings to be referred to is for the left leg.

The bone plate 1 is smaller than a typical long plate and is medium-sized suitably adapted to the tibia A of a small patient. The bone plate 1 includes the shaft portion 2 extending in the longitudinal direction X and the head portion 3 arranged on the proximal side of the shaft portion 2. The shaft portion 2 is a portion to be disposed on the diaphyseal A1, which is located on the distal side of the cut portion C. The head portion 3 is a portion to be disposed on the epiphyseal A2, which is located on the proximal side of the cut portion C, is shorter than the shaft portion 2 in the longitudinal direction X, and protrudes at both ends in the lateral direction Y with respect to the shaft portion 2. For example, the head portion 3 is substantially elliptical or substantially egg-shaped. The dimension L of the bone plate 1 in the longitudinal direction X is 80 mm or more and 90 mm or less. The width dimension (maximum width) W of the head portion 3 in the lateral direction Y is 23 mm or more and 29 mm or less, and preferably 24 mm or more and 25 mm or less.

The shaft portion 2 has four screw holes 4a, 4b, 4c, and 4d, and the head portion 3 has four screw holes 5a, 5b, 5c, and 5d. The holes 4a to 4d and 5a to 5d are screw holes penetrating the bone plate 1 in the thickness direction from a front side 1a to a rear side 1a.

Each of the bone screws 11a to 11d and 12a to 12d has a long shaft portion having a male screw to be screwed into the tibia A, and a head connected to the base end of the shaft portion and having a male screw. The bone screws 11a to 11d and 12a to 12d are inserted into screw holes 4a to 4d and 5a to 5d, respectively, and the heads are fastened to the screw holes 4a to 4d and 5a to 5d.

The four screw holes 4a, 4b, 4c, and 4d in the shaft portion 2 are arranged at intervals in the longitudinal direction X. Hereinbelow, the screw holes 4a, 4b, 4c, and 4d are referred to as a first (#1) hole, a second (#2) hole, a third (#3) hole, and a fourth (#4) hole in order from the proximal side toward the distal side. The bone screws 11a, 11b, 11c, and 11d to be inserted into the holes 4a, 4b, 4c, and 4d, respectively, are referred to as a #1 screw, a #2 screw, a #3 screw, and a #4 screw.

As shown in Fig. 1A, the #2 hole 4b may be arranged on the posterior side with respect to the row of the other three holes 4a, 4c, and 4d. Because the distance between two adjacent screw holes affects the local strength of the bone plate 1, it is preferable that the distance be greater than or equal to a certain value. By disposing the #2 hole 4b on the posterior side, it is possible to provide the shaft portion 2, which is shorter than the conventional bone plate, with four screw holes 4a, 4b, 4b, and 4c, which are the same number as the holes in the conventional bone plate, while ensuring the necessary intervals between the holes 4a and 4b and between the holes 4c and 4d.

Instead of the #2 hole 4b, another hole 4a, 4c, or 4d may be disposed on the posterior side with respect to the row of the remaining three holes.

The four screw holes in the head portion 3 include an A hole 5a, a B hole 5b, a C hole 5c, and a D hole 5d, which are respectively disposed on the anterior side, the proximal side, the posterior side, and the distal side, and are arranged in a quadrangular shape (that is, disposed at four apexes of the quadrangle). Hereinbelow, the four bone screws 12a, 12b, 12c, and 12d to be inserted into the holes 5a, 5b, 5c, and 5d, respectively, are referred to as an A screw, a B screw, a C screw, and a D screw.

More specifically, the A hole (first screw hole) 5a is disposed on the most anterior side among the four holes 5a, 5b, 5c, and 5d. The C hole (second screw hole) 5c is disposed posterior to the A hole 5a, at a certain distance therefrom in the lateral direction Y. The B and D holes 5b and 5d are located between the A hole 5a and the C hole 5c in the lateral direction Y. The B hole (third screw hole) 5b is disposed further on the proximal side than the A hole 5a and the C hole 5c, and the D hole (fourth screw hole) 5d is disposed further on the distal side than the A hole 5a and the C hole 5c.

The amount by which the A hole 5a is shifted to distal side with respect to the B hole 5b is greater than the amount by which the C hole 5c is of shifted to the distal side with respect to the B hole 5b. Hence, the A hole 5a is disposed on the distal side with respect to the C hole 5c. The D hole 5d is disposed on the posterior side with respect to the B hole 5b.

As shown in Fig. 2, in the front view as viewed in the direction substantially parallel to the center axis β of the B hole 5b, the distance d1 between the B hole 5b and a straight line L1 is preferably 2 mm or more and 7 mm or less, more preferably 5 mm or less, and ideally 5 mm. The straight line L1 passes through the center axis α of the A hole 5a and the center axis γ of the C hole 5c, and the distance d1 is the length of a perpendicular line drawn from the center axis β to the straight line L1.

The distance d2 between the A hole 5a and the C hole 5c is preferably 14 mm or more and 19 mm or less, more preferably 15 mm or less, and ideally 15 mm. The distance d2 is the distance between the axis α and the axis γ in the front view.

The distance d3 between the B hole 5b and the D hole 5d is preferably 11 mm or more and 15 mm or less, and ideally 12 mm. The distance d3 is the distance between the axis β and the center axis δ of the D hole 5d in the front view.

As shown in Fig. 3A, the three axes α, β, and γ are inclined in the directions toward each other as they are farther from the bone plate 1 in the insertion directions of the screws 12a, 12b, and 12c. Hence, the distal ends of the three screws 12, 12b, and 12c, whose heads are fastened to the holes 5a, 5b, and 5c, are arranged so as to be close to each other or in contact with each other. The insertion direction is a direction from the front side 1a toward the rear side 1b of the bone plate 1. More specifically, the axis α is inclined toward the posterior side along the insertion direction of the A screw 12a, and the axis γ is inclined toward the anterior side along the insertion direction of the C screw 12c. The axes α and γ may also be inclined toward the proximal side.

Furthermore, the axis δ is inclined with respect to the axis β in a direction toward the axis β as it is farther from the bone plate 1 in the insertion direction of the D screw 12d. Thus, the distal end of the D screw 5d, whose head portion is fastened to the hole 12d, enters the space surrounded by the distal ends of the three screws 12a, 12b, and 12c.

As shown in Fig. 3C, preferably, the distal end of the D screw 12d is in contact with the distal ends of at least two of the screws 12a, 12b, and 12c, so that at least three screws form a space truss structure. In Fig. 3C, the distal end of the D screw 12d is in contact with the distal ends of the B and C screws 12b and 12c. The distal end of the D screw 12d may be in contact with the A and B screws 12 and 12b, or may be in contact with the distal ends of the A, B, and C screws 12a, 12b, and 12c.

The distal ends of the screws 12a, 12b, 12c, and 12d forming the space truss structure are preferably disposed on the lateral surface of the epiphyseal A2 or in the vicinity thereof (see Fig. 3A). The lengths of the screws 12a, 12b, and 12c and the inclination angles of the axes α, β, γ, and δ are designed to realize the space truss structure, taking the dimensions of the tibia A of a small patient into consideration. For example, the lengths of the screws 12a, 12b, 12c, and 12d are 65 mm to 75 mm.

Next, a method of using the bone plate kit will be described.

As shown in Figs. 3A and 3B, in the OWHTO, the bone plate 1 is disposed on the medial surface of the tibia A across the cut portion C, and is fixed to the tibia A with the screws 11a to 11d and 12a to 12d. More specifically, the screws 11a, 11b, 11c, and 11d are inserted into the diaphyseal A1 through the holes 4a, 4b, 4c, and 4d, respectively, whereby the shaft portion 2 is fixed to the diaphyseal A1. Furthermore, the screws 12a, 12b, 12c, and 12d are inserted into the epiphyseal A2 through the screw holes 5a, 5b, 5c, and 5d, whereby the head portion 3 is fixed to the epiphyseal A2.

The distal ends of the four bone screws 12a, 12b, 12c, and 12d, which are arranged along the axes α, β, γ, and δ, respectively, are arranged close to or in contact with each other in the epiphyseal A2.

The tibia A of a small patient is smaller than the typical size tibia, and for example, the width of the epiphyseal A2 in the anteroposterior direction is about 85% of the width of the typical size epiphyseal. The width W and the overall length L of the head portion 3 of the bone plate 1 according to this embodiment are smaller than those of the conventional T-shaped bone plate 1'. Hence, as shown in Fig. 3A, in a state in which the distal end and the proximal end of the bone plate 1 are in contact with the medial surface, the clearance between the portion between the #1 hole 4a and the D hole 5d in the bone plate 1 and the medial surface is reduced. As described, the bone plate 1 fits well to the shape of the medial surface of the tibia A of a small patient, and thus, it is possible to reduce lifting of the bone plate 1 from the medial surface.

Fig. 4 shows a comparison between the head portion 3 of the bone plate 1 according to this embodiment and a head portion 3' of the conventional T-shaped bone plate 1', in which the head portions 3 and 3' are superposed so that the B holes 5b and 5b' coincide with each other. In one example of the conventional T-shaped bone plate 1', the width of the head portion 3' is 30 mm, and the overall length of the head portion 3' is100 mm long. In one design example of the bone plate 1 according to this embodiment, the width of the head portion 3 is approximately 25 mm, and the overall length of the bone plate 1 is approximately 90 mm. By reducing the width W of the head portion 3 in this way, it is possible to suppress lifting of the anterior portion of the head portion 3 from the medial surface of the epiphyseal A2. Furthermore, by reducing the length of the shaft portion 2, it is possible to suppress lifting of the shaft portion 2 from the medial surface.

According to this embodiment, the A hole 5a and the C hole 5c are arranged on the distal side with respect to the B hole 5b. This makes it possible to reduce the distance d2 between the A hole 5a and the C hole 5c and, thus, to reduce the width W of the head portions 3, while maintaining the same diameters of the holes 5a, 5b, 5c, and 5d and the same intervals between the holes 5a, 5b, 5c, and 5d as those in the conventional bone plate 1'.

For example, in the conventional bone plate 1', the diameters of the holes 5a' to 5d' are 6 mm, and the intervals between the holes 5a' and 5b' and between the holes 5b' and 5c' are 2 mm to 3 mm. Also in the bone plate 1 according to this embodiment, the diameters of the holes 5a to 5d are 6 mm, and the intervals between the holes 5a' and 5b' and between the holes 5b' and 5c' are 2 mm to 3 mm.

Because the fixing force of the bone plate 1 to the tibia A depends on the number and thickness of the bone screws, it is preferable that the number and diameter of the screw holes be the same as those of the conventional bone plate 1' to obtain the same fixing force as that of the conventional bone plate 1'. Furthermore, in order to maintain the strength of the bone plate 1, it is preferable that the intervals between the holes 5a, 5b, 5c, and 5d be the same as those of the conventional bone plate 1'. When the intervals between the screw holes are reduced, the strength of the bone plate may locally decrease.

Specifically, according to this embodiment, the above-described arrangement of the holes 5a, 5b, and 5c makes it possible to reduce the width W of the head portion 3 while achieving the same fixing force to the epiphyseal A2 and the same strength of the bone plate 1 as those of the conventional bone plate 1', in which the screw holes 5a', 5b', and 5c' are arranged in a line in the lateral direction Y.

Furthermore, according to this embodiment, the distal ends of at least three of the screws 12a, 12b, 12c, and 12d are in contact with each other on the posterior surface close to the hinge portion B or in the vicinity thereof to form a space truss structure, which is structurally stable. The screws 12a, 12b, 12c, and 12d support the load applied to the tibia A in the vicinity of the hinge portion B. Hence, it is possible to reduce the load applied to the hinge portion B, and to protect the hinge portion B from the load.

In addition, according to this embodiment, the distance d3 between the holes 5b and 5d is shorter than that of the conventional bone plate 1'. For example, the distance d3 is 15 mm in the conventional bone plate 1', and is 12 mm in the bone plate 1 according to this embodiment. By reducing the distance d3, even in the case of the small epiphyseal A2, the D screw 12d is less likely to interfere with an osteotomy surface E on the proximal side. Thus, a long bone screw, which provides a higher fixing force, can be used as the D screw 12d.

If the distance d3 between the holes 5b and 5d is equal to that of the conventional bone plate 1', the D screw 12d is likely to interfere with the osteotomy surface E in the small tibia A. To prevent the interference, a short D screw 12d that does not reach the osteotomy surface E needs to be used.

Furthermore, the axis δ is inclined toward the proximal side along the insertion direction of the D screw 12d. This makes it possible to use a longer D screw 12d without causing interference with the osteotomy surface E, and the D screw 12d can form a space truss structure with the A, B and C screws 12a, 12b, 12c.

According to this embodiment, because the D hole 5d is disposed on the posterior side with respect to the B hole 5b, the distance between the holes 5b and 5d in the longitudinal direction X is further reduced, and the axis δ of the D hole 5d is inclined toward the proximal side. This is advantageous when OWHTO is used in conjunction with meniscus centralization (arthroscopic centralization).

As shown in Fig. 5, centralization is a surgery for replacing the meniscus G that has prolapsed from the joint using a suture S. The suture S is fixed below the tibial articular surface with an anchor T. In the OWHTO using the conventional bone plate 1', as shown in Fig. 6A, the proximal end of the bone plate 1' is disposed near the articular surface H, and the B screw 12b' is inserted into the tibia A so as to be parallel to the articular surface H. Hence, the B screw 12b is likely to interfere with the suture S. In Fig. 5, reference sign I denotes the femur, reference sign J denotes the collateral ligament, and reference sign K denotes a cartilage defect.

According to this embodiment, the B screw 12d can be inserted into the tibia A at a position sufficiently away from the articular surface H while preventing the D screw 12b from interfering with the osteotomy surface E. For example, it is possible to secure a distance d4 of about 15 mm between the articular surface H and the B screw 12b (see Figs. 3A and 3B). Thus, it is possible to prevent the interference between the B screw 12b and the suture S.

Furthermore, according to this embodiment, because the A, B, and C holes 5a, 5b, and 5c are arranged in a triangle, the head portion 3 can be formed in a shape with less protrusion to the anterior side and the posterior side, such as a substantially elliptical shape or a substantially egg shape. This makes it possible to further suppress lifting of the anterior end and the posterior end of the head portion 3 when the head portion 3 is fixed to the epiphyseal A2 of a small patient. In particular, as a result of the A hole 5a being disposed on the distal side with respect to the C hole 5c, it is possible to effectively reduce the lifting of the anterior end of the head portion 3.

As shown in Fig. 4, in the conventional bone plate 1', the A, B, and C holes 5a', 5b', and 5c' are arranged in a line in the lateral direction. Thus, as shown by the hatched area in Fig. 4, the head portion 3' has such a shape that it significantly protrudes forward and rearward. Thus, the anterior end and the posterior end of the head portion 3' are likely to be lifted significantly.

Furthermore, according to this embodiment, it is possible to realize the rigidity of the bone plate 1 suitable for the bone fusion of the tibia A of a small patient.

A load F (see 3A in Fig. 1) applied to the knee articular surface H of the tibia A is distributed to the bone plate 1, an artificial bone (not shown) disposed in the cut portion C, the hinge portion B, and the fibula (not shown). In order to obtain high fixation of the tibia A, the bone plate 1 preferably has high rigidity. However, if the rigidity of the bone plate 1 is too high, correction loss may occur due to the screws 11a to 11d and 12a to 12d biting into the cancellous bone. Meanwhile, if the rigidity of the bone plate 1 is too low, the load applied to the artificial bone and the hinge portion B will be excessively large.

The rigidity of the bone plate 1 against the load F increases as the overall length L increases. According to this embodiment, the rigidity of the bone plate 1 can be optimized for the fixation of the small tibia A by reducing the overall length of the bone plate 1 to about 90 mm as compared with the conventional T-shaped bone plate 1'.

In this embodiment, the holes 5a to 5d in the head portion 3 are preferably arranged on the posterior side with respect to the row of the holes 4a, 4c, 4d in the shaft portion 2.

In Fig. 1A, the shaft portion 2 is bent in a direction in which it protrudes toward the anterior side in the vicinity of the #2 hole 4b. Thus, the holes 5a to 5d are disposed on the posterior side with respect to the row of the holes 4a, 4c, and 4d (see the one dot chain line). The bone plate 1 may be substantially L-shaped, in which the head portion 3 extends to the posterior side from the proximal end of the straight shaft portion 2.

In the conventional T-shaped bone plate 1', the vicinity of the A hole 5a', which is located on the extreme anterior side, is likely to be lifted from the surface of the epiphyseal A2.

According to the bone plate 1 having the above-described configuration, in a state in which the bone plate 1 is disposed on the medial surface of the diaphyseal A1 such that the row of the holes 4a, 4c, and 4d extends in the longitudinal direction of the diaphyseal A1, the head portion 3 is disposed on the posterior medial surface of the epiphyseal A2, and the A hole 5a is disposed further on the posterior side (see Fig. 3B ). With this structure, the lifting of the anterior portion of the head portion 3 can be further suppressed.

In the above embodiment, the quadrangular arrangement of the holes 5a, 5b, 5c, and 5d can be appropriately changed as long as the B hole 5b is arranged on the proximal side with respect to the A and C holes 5a and 5c, and the D hole 5d is arranged on the distal side with respect to the A and C holes 5a and 5c.

For example, the A hole 5a may be disposed at the same position as the C hole 5c in the longitudinal direction X, and the D hole 5d may be disposed at the same position as the B hole 5b in the lateral direction Y. Such an arrangement can also provide the effect of reducing the width W of the head portion 3.

In the above embodiment, the number of the screw holes in the shaft portion 2 is four. Instead, the number of the screw holes may be two, three, or five or more.

In the above embodiment, the bone plate 1 for the OWHTO has been described. However, the application of the bone plate 1 according to this embodiment is not limited to the tibia A and the OWHTO, and the bone plate 1 may be used for bones other than the tibia A and bone surgeries other than the OWHTO. For example, the bone plate 1 may be used for internal fixation of a fractured bone.

### {Reference Signs List}

1 Bone plate
2 Shaft portion
3 Head portion
4a, 4b, 4c, 4d Screw hole
5a A hole (first screw hole)
5b B hole (third screw hole)
5c C hole (second screw hole)
5d D hole (fourth screw hole)
12a, 12b, 12c, 12d Bone screw
A Tibia
A1 Diaphyseal
A2 Epiphyseal
d1, d2, d3 Distance
X Longitudinal direction
Y Lateral direction
α, β, γ, δ Center axis

## Claims

1. A bone plate to be disposed on a medial surface of a tibia, the bone plate having a shape of a band having a longitudinal direction arranged along a longitudinal direction of the tibia and a lateral direction arranged along an anteroposterior direction of the tibia, having a proximal side and a distal side corresponding to a proximal side and a distal side of the tibia, respectively, and comprising:
a shaft portion extending in the longitudinal direction; and
a head portion disposed on a proximal side of the shaft portion,
wherein the head portion has four screw holes arranged in a quadrangular shape, and
the four screw holes include:
a first screw hole and a second screw hole arranged in the lateral direction with a space therebetween;
a third screw hole located between the first screw hole and the second screw hole in the lateral direction and disposed further on the proximal side than the first and second screw holes; and
a fourth screw hole located between the first screw hole and the second screw hole in the lateral direction and disposed further on the distal side than the first and second screw holes.

2. The bone plate according to Claim 1, wherein
a dimension of the head portion in the lateral direction is 25 mm or less,
a dimension of the bone plate in the longitudinal direction is 90 mm or less,
a distance between the third screw hole and a straight line passing through a center axis of the first screw hole and a center axis of the second screw hole is 2 mm or more and 7 mm or less,
a distance between the first screw hole and the second screw hole is 14 mm or more and 19 mm or less, and
a distance between the third screw hole and the fourth screw hole is 11 mm or more and 15 mm or less.

3. The bone plate according to Claim 1, wherein
the first screw hole is disposed on the distal side with respect to the second screw hole, and
the fourth screw hole is disposed on a posterior side with respect to the third screw hole.

4. The bone plate according to Claim 1, wherein
the shaft portion has four screw holes arranged in the longitudinal direction, and
one of the four screw holes is disposed on a posterior side with respect to a row of the other three screw holes.

5. A bone plate kit comprising:
the bone plate according to Claim 1; and
four bone screws to be inserted into the four screw holes,
wherein center axes of the four screw holes are inclined with respect to each other in directions toward each other as the center axes are farther from the bone plate in insertion directions of the bone screws, and
at least three of the four bone screws passing through the four screw holes contact each other at distal ends thereof to form a space truss structure.
